# EUROPÄISCHE PATENTANMELDUNG

(11) **EP 3 656 288 A1**
(43) Veröffentlichungstag der Anmeldung: **27.05.2020**
(21) Anmeldenummer: 19207506.7
(22) Anmeldetag: 06.11.2019
(51) Int. Cl.: A61B 3/16, A61B 3/00, A61B 3/135, A61B 1/00, A61B 1/227

(54) **PRÜFVERFAHREN FÜR DISPOSABLE**

(30) Priorität: 21.11.2018 CH 14362018
(71) Anmelder: HAAG-STREIT AG, 3098 Köniz (CH)
(72) Erfinder: Breitenstein, Jörg, 3052 Zollikofen (CH); Zumkehr, Frank, 3052 Zollikofen (CH)
(74) Vertreter: Keller & Partner Patentanwälte AG

(57) **Zusammenfassung**

In einem Verfahren zum Prüfen eines Disposables (20) an einem medizinischen Untersuchungsgerät, insbesondere an einem Tonometer (10), wobei das medizinische Untersuchungsgerät eine Disposable-Haltevorrichtung (15) umfasst, an welchem ein Disposable (20) gehalten und insbesondere zur applanationstonometrischen Messung mit einem Patientenauge in Kontakt bringbar ist und welche eine Erfassungseinrichtung (11, 12; 122) umfasst, womit eine physikalische Eigenschaft des Disposables (20) gemessen werden kann, wird mittels der Erfassungseinrichtung (11, 12; 122) ein Messwert der physikalischen Eigenschaft des Disposables ermittelt.

## Beschreibung

### Technisches Gebiet

Die Erfindung betrifft ein Verfahren zum Prüfen eines Disposables an einem medizinischen Untersuchungsgerät, insbesondere an einem Tonometer, wobei das medizinische Untersuchungsgerät eine Disposable-Haltevorrichtung umfasst, an welchem ein Disposable gehalten und insbesondere zur applanationstonometrischen Messung mit einem Patientenauge in Kontakt bringbar ist sowie eine Erfassungseinrichtung umfasst, womit eine physikalische Eigenschaft des Disposables gemessen werden kann.

### Stand der Technik

Medizinische Untersuchungsgeräte unterliegen strengen Hygienevorschriften. Damit sollen Krankheitsübertragungen, Infektionen etc. in Spitälern wie auch bei Ärzten bestmöglich vermieden werden. Eine grosse Gefahr für Kontaminationen stellen Bestandteile von Vorrichtungen, insbesondere von medizinischen Untersuchungsgeräten, dar, welche in direkten Kontakt mit dem Patienten kommen.

Um das Risiko einer Kontamination zu vermindern, werden solche Bestandteile vermehrt als sogenannte Disposables ausgebildet. Dabei handelt es sich um Bestandteile, respektive Verbrauchsmaterial als Zubehör für ein medizinisches Untersuchungsgerät, welche typischerweise nur einmal verwendet werden. Ein Disposable kann zum Beispiel eine Schutzhülle für einen Teil eines Untersuchungsgeräts sein, welches während einer Untersuchung oder einer Behandlung in Kontakt mit dem Patienten gebracht wird. Weiter kann es sich um einen Bestandteil handeln, welcher selbst zur Durchführung der Messung oder der Behandlung notwendig ist.

Im Falle eines Tonometers kann der Bestandteil ein Prüfkörper sein, welcher zur Applanation gegen die Hornhaut eines Auges gedrückt wird. Weiter kann der Bestandteil auch ein Kontaktglas oder ein Teil eines Kontaktglases für die Funduskopie sein. Aber auch bei einem Otoskop kann ein Disposable vorgesehen sein, welches als Kappe ausgebildet sein kann und damit einen transparenten Boden als Sichtfenster umfasst.

Die US 4,922,914 (Elizabeth O. Segal et al.) offenbart zum Beispiel eine Schutzhülse in Form eines Disposables für ein Goldmann-Tonometer. Mit der Schutzhülse für den Tonometerkopf kann der direkte Kontakt zwischen Tonometerkopf und Patient vermieden werden. Die Schutzhülse wird für jeden Patienten ersetzt. Die Schutzhülse umfasst weiter an der Kontaktfläche eine abziehbare Klebefolie zum Schutz der Kontaktfläche.

Die bekannten medizinischen Untersuchungsgeräte, insbesondere Tonometer, haben den Nachteil, dass der Anwender zum Beispiel gegebenenfalls vergessen kann, dass Disposable zwischen zwei Untersuchungen durch ein neues Disposable auszutauschen. Damit kann eine Übertragung von Erregern des ersten Patienten an den zweiten Patienten erfolgen. Weiter können Disposables eingesetzt werden, welche unter Umständen für die Vorrichtung nicht geeignet sind, womit die Messresultate beeinträchtigt werden können. Gesamthaft ist damit der Einsatz von Disposables mit vielen Risiken verbunden. Die Bekannten medizinischen Untersuchungsgeräte, insbesondere Tonometer, tragen, trotz dem Einsatz von Disposables, der Sicherheit des Patienten noch zu wenig Rechnung.

### Darstellung der Erfindung

Aufgabe der Erfindung ist es, eine dem eingangs genannten technischen Gebiet zugehörendes Verfahren zu schaffen, womit der Einsatz eines medizinischen Untersuchungsgeräts sicherer gestaltet werden kann.

Die Lösung der Aufgabe ist durch die Merkmale des Anspruchs 1 definiert. Gemäss der Erfindung wird mittels der Erfassungseinrichtung ein Messwert der physikalischen Eigenschaft des Disposables ermittelt.

Damit wird ermöglicht, dass Messwerte erfasst werden können, welche dem Disposable zugeordnet werden können. Die Messwerte können anschliessend eingesetzt werden, um die Sicherheit bei einem Einsatz des medizinischen Untersuchungsgeräts, insbesondere des Tonometers zu erhöhen.

Das medizinische Untersuchungsgerät kann prinzipiell beliebig gewählt sein, sofern ein Disposable einsetzbar ist. Typischerweise ist das medizinische Untersuchungsgerät als ein Gerät ausgebildet, welches zur Durchführung einer Messung in Kontakt mit dem Patienten gebracht wird. In einer besonders bevorzugten Ausführungsform handelt es sich bei dem medizinischen Untersuchungsgerät um ein Tonometer, insbesondere um ein Tonometer zur Applanationsmessung. In diesem Fall handelt es sich bei dem Disposable um einen Prüfkörper respektive um eine Schutzhülle für einen Prüfkörper. Das Disposable wird somit zur Applanation gegen die Hornhaut eines Auges gedrückt.

In Varianten kann das Disposable auch ein Bestandteil eines Kontaktglases oder ein Teil eines Kontaktglases für die Funduskopie sein. Weiter kann das Disposable auch als Schutzhülle eines Otoskopes ausgebildet sein.

Die Erfassungseinrichtung kann ebenfalls in weiten Bereichen beliebig ausgebildet sein und hängt im Wesentlichen von der Art der zu erfassenden physikalischen Eigenschaft ab.

Die Erfassungseinrichtung kann zum Beispiel einen Bildsensor umfassen, womit ein optischer Messwert des Disposable ermittelt werden kann. Der Bildsensor kann zum Beispiel als CCD-Sensor, CMOS-Sensor oder andere dem Fachmann bekannte Sensoren umfassen. Der Sensor kann insbesondere unterschiedliche Sensortypen mit gegebenenfalls unterschiedlichen Aufnahmespektren und Auflösungen umfassen.

Die Erfassungseinrichtung kann jedoch auch weitere Sensoren oder Mittel umfassen, womit eine physikalische Eigenschaft des Disposables ermittelt werden kann (siehe dazu weiter unten).

Vorzugsweise wird aufgrund des Messwertes dem medizinischen Untersuchungsgerät, insbesondere dem Tonometer, ein Betriebszustand zugeordnet. Damit kann insbesondere aufgrund des Messwertes entschieden werden, ob das medizinische Messgerät, insbesondere das Tonometer, betriebsbereit ist oder nicht. Damit kann ein sicheres Messverfahren mit dem medizinischen Untersuchungsgerät geschaffen werden. Sofern aufgrund des Messwertes ermittelt wird, dass das Untersuchungsgerät nicht betriebsbereit ist, kann zum Beispiel ein Warnsignal ausgegeben werden. Weiter kann, alternativ oder zusätzlich, das Auslösen einer Messung elektronisch unterbunden werden. Weiter kann auch lediglich in den Messwerten ein Vermerk über den Betriebszustand zugeordnet, insbesondere abgespeichert respektive gedruckt werden.

In Varianten oder zusätzlich können die Messwerte gespeichert werden. Zum Beispiel kann eine Chargennummer oder dergleichen ausgelesen und dem Messergebnis der medizinischen Untersuchung zugeordnet werden. Damit kann zu einem späteren Zeitpunkt rückverfolgt werden, mit welchen Disposables ein Patient untersucht wurde. Damit kann zum Beispiel bei einem Rückruf einer fehlerhaften Charge Dispoables einfach ermittelt werden, bei welchen Patienten eine medizinische Untersuchung wiederholt werden muss.

Vorzugsweise umfasst die physikalische Eigenschaft eine Eigenfrequenz des Disposables und/oder eines Disposable umfassenden Teils des medizinischen Untersuchungsgeräts, insbesondere des Tonometers.

Unter dem Begriff "a und/oder b" werden vorliegend eine der Mengen {a}, {b} respektive {a,b} verstanden.

Die Eigenfrequenz dient dabei zur Identifikation des Disposables. Sofern die gemessene Eigenfrequenz mit einer dem Disposable zugeordneten Eigenfrequenz übereinstimmt, kann dem Untersuchungsgerät, insbesondere dem Tonometer, ein Betriebszustand zugeordnet werden, welcher eine Durchführung der Messung erlaubt. Sofern eine Eigenfrequenz ermittelt wird, welche ausserhalb eines zulässigen Frequenz-Intervalls liegt, kann dem Untersuchungsgerät ein Betriebszustand zugeordnet werden, welcher keine Durchführung einer Messung erlaubt. Alternativ oder zusätzlich kann die ermittelte Eigenfrequenz respektive eine dazu korrelierende Information zusammen mit dem Messergebnis des Untersuchungsgeräts, insbesondere zum Beispiel dem Applanationsdruck, gespeichert oder ausgegeben werden.

Die Eigenfrequenz muss dabei nicht zwingend vom Disposable als solches ermittelt werden. Stattdessen können auch mehrere Bestandteile des medizinischen Untersuchungsgeräts zusammen mit dem Disposable in Schwingung versetzt werden, um eine aussagekräftige Eigenfrequenz zu ermitteln. Dabei beeinflusst das Disposable die Eigenfrequenz des schwingenden Systems vorzugsweise hinreichend, so dass ein fehlendes Disposable, ein nicht korrekt montiertes Disposable oder ein nicht kompatibles Disposable identifiziert werden kann.

Durch das Erzeugen einer Schwingung kann zudem geprüft werden, ob das Disposable auf der Disposable-Haltevorrichtung genügend fest montiert ist, so dass es auch während der Untersuchung sich nicht von der Disposable-Haltevorrichtung lösen kann.

In Varianten kann auf die Bestimmung der Eigenfreqeunz auch verzichtet werden. In diesem Fall kann das Disposable und/oder einen das Disposable umfassenden Teil des medizinischen Untersuchungsgeräts in eine andere Schwingung oder eine sonstige Bewegung versetzt werden, womit Rückschlüsse auf das Disposable erhalten werden können.

Vorzugsweise wird zur Ermittlung der Eigenfrequenz das Disposables und/oder ein das Disposable umfassender Teil des medizinischen Untersuchungsgeräts durch eine Vorrichtung zur Erzeugung einer Applanationskraft, in eine Schwingung versetzt. Damit umfasst das medizinische Untersuchungsgerät vorzugsweise ein Tonometer, welches einen Aktuator zur Ausübung einer Applanationskraft umfasst. Dieser Aktuator kann unterschiedlich ausgebildet sein.

In Varianten kann eine separate Vorrichtung vorgesehen sein, womit das Disposable respektive das Disposable zusammen mit der Disposable-Haltevorrichtung in eine Schwingung versetzt werden kann.

Vorzugsweise wird die Schwingung elektromagnetisch erzeugt. Dazu umfasst der Aktuator einen Elektromagneten, womit auf die Disposable-Haltevorrichtung mit dem Disposable eine Kraft ausgeübt werden kann. Der Einsatz eines Elektromagneten hat den Vorteil, dass nur wenige bewegte Teile benötigt werden, insbesondere kann im Wesentlichen eine Führung für die Disposable-Haltevorrichtung und einen die Disposable-Haltevorrichtung tragenden magnetische Kern ausreichend sein. Natürlich kann alternativ dazu auch der Kern ortsfest sein und die Disposable-Haltevorrichtung mit einer mit dem magnetischen Kern zusammen wirkenden Spule verbunden sein.

Zur Ermittlung der Eigenfrequenz kann zum Beispiel anhand eines Verlaufs der magnetischen Feldstärke, der aufgenommenen Stromstärke, der Anzahl Windungen der Spule etc. direkt ermittelt werden. Weiter kann die Eigenfrequenz auch über einen Positionssensor ermittelt werden, mit welchem eine Frequenz des Disposables ermittelt werden kann.

Alternativ kann der Aktuator auch mechanisch, durch einen Schrittmotor oder dergleichen bereitgestellt werden, womit die Disposable-Haltevorrichtung mit einer Kraft beaufschlagt werden kann. Weiter kann auch ein hydraulisches oder pneumatisches System mit Druckzylindern zur Ausübung des Applanationsdruckes vorgesehen sein. Der Aktuator kann auch mit oder gegen eine Federkraft wirken. Weiter kann der Aktuator auch Dämpfungselemente zum Dämpfen der Bewegung des Disposables umfassen. Dem Fachmann sind weitere mögliche Techniken bekannt, womit die Disposable-Haltevorrichtung zur Ausübung eines Applanationsdruckes mit einer Kraft beaufschlagt werden kann.

Vorzugsweise wird anhand des Messwertes der Eigenfrequenz mindestens eines der folgenden Merkmale bestimmt:
- eine Ausrichtung des Disposables am medizinischen Untersuchungsgerät;
- eine Masse des Disposables;
- eine Massenverteilung, insbesondere ein Schwerpunkt des Disposables.

Damit kann ermittelt werden, ob es sich bei dem Disposable einerseits um ein zulässiges Disposable handelt. Dies ist wichtig, um die bestmöglichen Messresultate durch das medizinische Untersuchungsgerät erzielen zu können. Weiter wird damit sichergestellt, dass der Patient nicht durch ungeprüfte Disposables gefährdet wird.

Weiter kann damit ermittelt werden, ob das Disposable richtig positioniert ist. Sofern das Disposable kappenartig ausgebildet ist, kann damit zum Beispiel festgestellt werden, ob das Disposable bis zum Anschlag auf die Disposable-Haltevorrichtung aufgestülpt ist.

Da die Disposable-Haltevorrichtung und deren Schwingungsverhalten bekannt ist, kann aufgrund der Änderungen des Schwingungsverhaltens zum Beispiel auf einen Schwerpunkt des Disposables etc. geschlossen werden. Damit kann wiederum ermittelt werden, ob es sich um ein zulässiges und korrekt positioniertes Disposable handelt.

Dem Fachmann ist klar, dass nicht zwingend zwischen korrekter Positionierung und korrektem Disposable unterschieden werden muss. Im Verfahren reicht es grundsätzlich aus, wenn festgestellt werden kann, ob das Untersuchungsgerät betriebsbereit ist oder nicht.

In Varianten können statt der obigen Merkmale auch andere Merkmale des Disposables ermittelt werden, zum Beispiel ein Dämpfungsverhalten oder dergleichen.

In einer weiteren bevorzugten Ausführungsform umfasst die physikalische Eigenschaft ein optisches Reflexionsverhalten des Disposables. Das optische Reflexionsverhalten wird vorzugsweise durch einen Bildsensor, insbesondere durch einen Bildsensor einer Spaltlampe ermittelt. In Varianten können auch separate Sensoren vorgesehen sein, welche durch das Tonometer umfasst sind. Im Verfahren wird vorzugsweise vom Disposable reflektiertes Licht mit dem Bildsensor erfasst und elektronisch ausgewertet. Anschliessend können die ausgewerteten Daten mit Referenzwerten verglichen werden, Sofern die ausgewerteten Daten in einem vorgegebenen Referenzbereich liegen, kann dem Untersuchungsgerät ein Betriebszustand zugewiesen werden, welcher eine Durchführung einer Messung erlaubt. Anderseits kann zum Beispiel eine Warnung herausgegeben werden oder das Untersuchungsgerät für Untersuchungen zum Beispiel elektronisch blockiert werden.

In Varianten oder zusätzlich können auch weitere physikalische Eigenschaften des Disposables, insbesondere direkt oder indirekt gemessen werden. Sofern das Disposable zumindest geringfügig elektrische Leitfähig ist, kann zum Beispiel ein Widerstand im Disposable ermittelt werden. Dem Fachmann sind weitere Möglichkeiten bekannt.

Bevorzugt umfasst das optische Reflexionsverhalten ein optisches Erfassen von Daten des Disposables. Bei den Daten kann es sich um verschiedene, insbesondere um eine oder mehrere der folgenden Informationen handeln:
- Hersteller, z.B. Firma, Herstellerland etc;
- Typenbezeichnung;
- Chargennummer;
- Herstellungsdatum;
- Verfalldatum;
- Liste der dafür zugelassenen Tonometer;
- etc.

Die Daten erlauben es noch vor der Durchführung einer Untersuchung mit dem Untersuchungsgerät zu ermitteln, ob das Disposable für das vorliegende Tonometer zulässig ist. Dies kann durch Vergleich einer Typennummer des Disposables mit einer Positivliste des Tonometers erfolgen. Weiter kann das Disposable bereits die zulässigen Tonometer in den Daten erfasst haben. Weiter kann anhand des Herstellungsdatums respektive eines Verfalldatums entschieden werden, ob das Disposable noch verwendet werden darf. Eine oder mehrere der obig aufgelisteten Informationen können dem Messresultat des Untersuchungsgeräts zugeordnet werden. Dazu können die Informationen zusammen mit dem Messresultat, insbesondere des Messresultats des Tonometers, abgespeichert oder ausgegeben werden. Dem Fachmann sind weitere Informationen bekannt, welche zusätzlich auf dem Disposable festgehalten werden können. Zudem können die Informationen nach der Erfassung durch die Erfassungseinrichtung auch anderweitig verwendet werden.

In Varianten kann auf die Erfassung von Daten des Disposables auch verzichtet werden.

Bevorzugt umfassen die Daten des Disposables einen Code, insbesondere einen zweidimensionalen Code wie zum Beispiel einem QR-Code oder einen eindimensionalen Code, wie zum Beispiel einen Barcode, wobei mit dem medizinischen Untersuchungsgerät der Code erfasst wird. Damit können mit geringem Platzbedarf viele Informationen auf dem Disposable untergebracht werden. Dies ist insbesondere von Vorteil, da ein Disposable relativ klein ist. Statt des QR-codes oder des Barcodes können auch andere dem Fachmann bekannte Codierungen verwendet werden. Der Code kann direkt in die entsprechenden Informationen wie Chargennummer, Hersteller etc. umwandelbar sein. Alternativ kann der Code auch lediglich einen Pointer für eine Liste umfassen, womit zum Beispiel mit einer Nummer des Codes auf eine Zeilennummer einer Liste verwiesen wird. Damit kann der Platzbedarf weiter verringert werden. Dem Fachmann sind auch dazu weitere Möglichkeiten bekannt.

In Varianten kann der Code auch Buchstaben und Zahlen umfassen. Der Code muss nicht zwingend verschlüsselt sein, sondern kann auch als Klartext vorliegen. Dem Fachmann sind weitere Codierungstechniken bekannt.

Ein Prüfkörper zur Verwendung in einem Tonometer umfasst eine Kontaktfläche zum Kontaktieren der Hornhaut und einen Code, welcher insbesondere durch eine Erfassungseinrichtung des Untersuchungsgeräts, insbesondere des Tonometers erfassbar ist.

Vorzugsweise umfasst die Kontaktfläche einen Code, welcher durch eine Erfassungseinrichtung des Untersuchungsgeräts, insbesondere des Tonometers erfassbar ist.

In Varianten kann der Code auch ausserhalb der Kontaktfläche angeordnet sein (siehe unten).

Bevorzugt ist der Code zentral auf der Kontaktfläche, insbesondere auf einer Fläche kleiner als 3x3 mm, besonders bevorzugt kleiner als 2x2 mm, insbesondere bevorzugt auf ungefähr 1.5x1.5 mm dargestellt. Bei der tonometrischen Messung wird typischerweise ein Druck auf das Disposable ausgeführt, bis eine vorgegebene Applanationsfläche erreicht ist. Die Form der Applanationsfläche ist im Idealfall kreisförmig, kann aber in der Praxis auch oval oder anderweitig ausfallen. In nahezu jedem Fall ist jedoch ein zentraler Bereich des Prüfkörpers, insbesondere des Disposables, innerhalb einer Applanationsfläche, so dass in diesem zentralen Bereich Informationen zum Disposable wiedergegeben werden können. Die maximal zur Verfügung stehende Fläche, hängt damit von der Art des Messverfahrens ab. Zum Beispiel wird bei einer Messung nach Goldmann eine Applanationsfläche mit einem Durchmesser von 3.06 mm angestrebt. Damit kann zum Beispiel ein Innkreisquadrat eine Kantenlänge von 2.16 mm aufweisen. Aufgrund von möglichen Abweichungen von der idealen Kreisform wird in der Praxis bei einem Verfahren nach Goldmann vorzugsweise ein Bereich mit einer Kantenlänge von weniger als 2 mm für den Code verwendet, insbesondere ein Bereich mit einer Kantenlänge von ungefähr 1.5 mm.

Der Bereich kann grundsätzlich beliebig klein gewählt werden. Der mögliche Informationsgehalt des Bereichs hängt dabei von der Art der Codierung und von der Auflösung der Erfassungsvorrichtung ab.

Der Bereich kann natürlich auch andere Aussenkonturen aufweisen (rechteckig, rund etc.). Je nach Art des Messverfahrens kann der zentrale Bereich für den Code auch grösser als 3x3 mm gewählt werden.

In einer weiteren bevorzugten Ausführungsform ist der Code kreisringförmig ausgebildet und in einem zur Kontaktfläche peripheren Bereich angeordnet. Damit kann der Code um den Bereich angeordnet werden, welcher für die Messung relevant ist und trotzdem durch die Erfassungsvorrichtung erfassbar sein.

Bevorzugt werden Verunreinigungen und/oder Beschädigungen des Disposables aufgrund des optischen Reflexionsverhaltens ermittelt. Auch wenn es sich um ein korrekt positioniertes und zulässiges Disposable handelt, kann eine Untersuchung durch Verunreinigungen beeinträchtigt werden. Über das Reflexionsverhalten kann nun festgestellt werden, ob das Disposable Verunreinigungen aufweist und kann anhand der Messergebnisse dem medizinischen Untersuchungsgerät, insbesondere dem Tonometer, einen Betriebszustand zuordnen. Weiter kann mit der Erfassungseinrichtung auch eine allfällige Beschädigung des Disposables festgestellt werden, worauf entsprechend ein Betriebszustand zugeordnet werden kann. In der bevorzugten Ausführungsform wird mit der Erfassungseinrichtung der gesamte für die Messung relevante Bereich des Disposables erfasst, so dass im Wesentlichen alle Beschädigungen oder Verunreinigungen des Disposables, welche eine medizinische Untersuchung beeinträchtigen können, erfasst werden. Dies ist insbesondere dann der Fall, wenn als Erfassungseinrichtung ein Bildsensor eingesetzt wird, mit welchem zugleich die Applanationsfläche vermessen wird (siehe unten).

Alternativ oder zusätzlich können auch weitere Teile des Tonometers auf Unversehrtheit und Verschmutzungen geprüft werden. Insbesondere kann die Disposable-Haltevorrichtung in einem lichtleitenden Bereich auf Beschädigungen und/oder Verunreinigungen überprüft werden. In Varianten kann auf die Detektion von Verunreinigungen auch verzichtet werden.

Vorzugsweise wird das optische Reflexionsverhalten mit einem Bildsensor des Untersuchungsgeräts und/oder einer Spaltlampe ermittelt. Der oder die Bildsensoren sind Teil der Erfassungseinrichtung. Damit ist vorzugsweise der Bildsensor durch ein Untersuchungsgerät umfasst. In der bevorzugten Ausführungsform ist der Bildsensor durch eine Spaltlampe umfasst und das medizinische Untersuchungsgerät umfasst sowohl eine Spaltlampe als auch ein Tonometer. Das Tonometer kann auch als autonomes Gerät vorliegen, welches zum Beispiel bei Bedarf an die Spaltlampe gekoppelt werden kann, so dass eine Bildauswertung über die entsprechende Einrichtung der Spaltlampe erfolgen kann. Das Tonometer kann auch fest mit der Spaltlampe verbunden sein und zum Beispiel bei Bedarf in den Strahlengang der Spaltlampe einschwenkbar sein. Dem Fachmann sind weitere Varianten bekannt.

Besonders bevorzugt wird in der Verwendung des Tonometers, mit dem Bildsensor der Spaltlampe eine Applanationsfläche elektronisch vermessen. Einerseits kann damit eine besonders präzise Messung erreicht werden. Andererseits können damit mit demselben Bildsensor unterschiedliche Untersuchungen durchgeführt werden, womit einerseits die Vorrichtung und damit auch das Verfahren kostengünstig sein kann. Weiter kann damit für die tonometrische Untersuchung Licht ausserhalb des sichtbaren Lichtspektrums eingesetzt werden, womit für den Patienten eine angenehmere Augenuntersuchung erreicht werden kann. Zudem kann damit eine tonometrische Messung ohne Fluorescein durchgeführt werden. Die Kontaktfläche kann über ein Tränenfilmdepot zwischen dem Disposable und der Hornhaut elektronisch erfasst werden.

Das Tonometer kann dazu derart ausgebildet sein, dass mit einem Aktuator das Disposable gegen die Hornhaut eines Auges gedrückt wird. Der Aktuator wird dabei vorzugsweise anhand der Applanationsfläche, welche über den Bildsensor ermittelt wird, gesteuert. Dazu kann ein durch den Aktuator erzeugter Applanationsdruck beim Erreichen einer vorgegebenen Applanationsfläche abgespeichert oder ausgegeben werden. Dem Fachmann sind entsprechende Verfahren bekannt.

In Varianten kann die Erfassungseinrichtung auch durch ein anderes Untersuchungsgerät umfasst sein. Die Erfassungseinrichtung als separate Vorrichtung vorliegen oder kann Teil des Tonometers sein.

Eine Vorrichtung zur Durchführung eines Verfahrens nach einem der Ansprüche 1 bis 11, umfasst ein Tonometer mit einer Disposable-Haltevorrichtung, wobei die Disposable-Haltevorrichtung ein Ringbeleuchtung umfasst, welche als Anschlag für einen Rand eines kappenförmigen Disposables ausgebildet ist. Damit kann in einem Verfahren über die Ringbeleuchtung Licht durch die Wand des kappenförmigen Disposable zur Kontaktfläche geleitet werden. Im Bereich der Applanation wird die Kontaktfläche dunkel, wodurchmit einer Erfassungseinrichtung, insbesondere einem Fotosensor, die Applanationsfläche erfasst und ausgewertet werden kann.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung einer Seitenansicht eines Tonometerkopfes;
- Fig. 2: eine Darstellung gemäss Figur 1 mit aufgesetztem Disposable;
- Fig. 3: eine Darstellung gemäss Figur 2, wobei das Disposable als Schnittbild dargestellt ist;
- Fig. 4: eine schematische Seitenansicht einer Spaltlampe umfassend ein Tonometer;
- Fig. 5: eine schematische Draufsicht auf ein Disposable mit einem zentral auf der Kontaktfläche aufgebrachtem Code; sowie
- Fig. 6: eine schematische Draufsicht auf ein Disposable mit einem im Randbereich der Kontaktfläche aufgebrachtem Code.

Grundsätzlich sind in den Figuren gleiche Teile mit gleichen Bezugszeichen versehen.

### Wege zur Ausführung der Erfindung

Generell sind die nachfolgend erläuterten Abbildungen stark vereinfacht. Auf detaillierte Darstellungen von optischen Bauteile, insbesondere von Linsensystemen zur Vergrösserung und Fokusierung, Filter etc. wird bewusst verzichtet, da dem Fachmann deren Aufbau hinreichend bekannt ist.

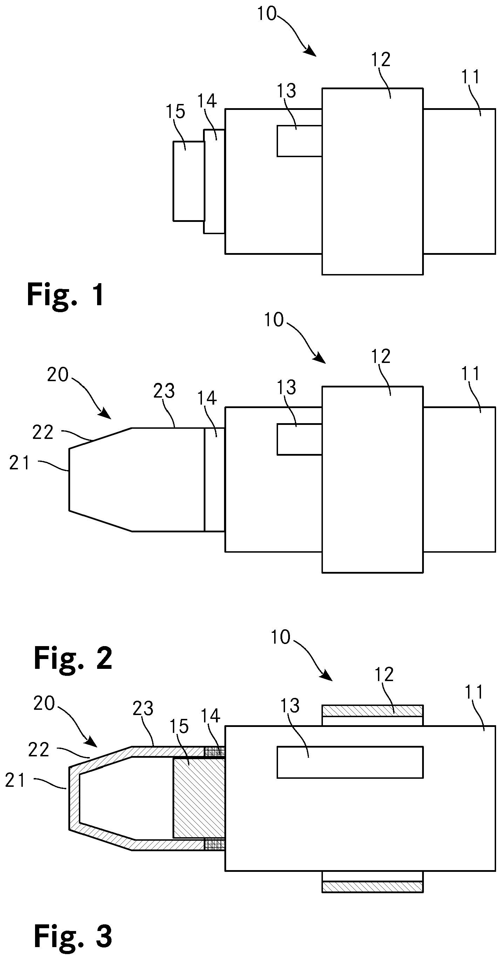

Die Figur 1 zeigt eine schematische Darstellung einer Seitenansicht eines Tonometerkopfes 10. Der Tonometerkopf 10 umfasst einen Tonometerkörper 11. Dieser weist eine Ringbeleuchtung 14 und einen Sockel 15 zur Aufnahme eines als Disposable ausgebildeten Prüfkörpers auf.

Der Tonometerkörper 11 ist in einer Führungsvorrichtung 12 längsverschieblich geführt. Die Führungsvorrichtung 12 umfasst eine elektrisch ansteuerbare Spule zum Erzeugen eines Magnetfeldes, während der Tonometerkörper 11 ein nicht näher dargestelltes magnetisches Element umfasst. Wird nun in der Spule ein Magnetfeld erzeugt, wird der Tonometerkörper 11 in der Führungsvorrichtung 12 mit einer Kraft beaufschlagt und in der Längsrichtung verschoben.

Um die Lage des Tonometerkörpers 11 relativ zur Führungsvorrichtung 12 bestimmen zu können, umfasst der Tonometerkörper 11 einen Positionssensor 13. Solche Sensoren sind dem Fachmann bekannt.

Die Figur 2 zeigt eine Darstellung gemäss Figur 1 mit aufgesetztem Disposable 20. Das Disposable 20 ist vorliegend kappenförmig ausgebildet. Zur besseren Illustration zeigt die Figur 3 zusätzlich eine Darstellung gemäss Figur 2, wobei das Disposable 20 sowie die Führungsvorrichtung 12 als Schnittbild dargestellt sind.

Das Disposable 20 umfasst eine Kontaktfläche 21, welche im Verfahren zum Ermitteln eines Innendruckes eines Auges gegen die Hornhaut gedrückt wird. An die Kontaktfläche 21 schliesst sich ein kreiskegelstumpfmantelförmiger Bereich 22 an, welcher in einen kreiszylindermantelförmigen Bereich 23 übergeht. Hergestellt ist es vorliegend aus einem klaren Kunststoff, insbesondere aus Acrylglas respektive PMMA (Polymethylmethacrylat). Acrylglas ist insbesondere zu bevorzugen weil es Licht besonders gut transmittiert. Dem Fachmann ist aber klar, dass andere Materialien auch eingesetzt werden können.

Der Tonometerkopf 11 umfasst einen Sockel 15, auf weichen das Disposable 20 aufgesteckt wird. Der Sockel 15 umfasst einen optischen Kanal, welcher in Längsrichtung den Tonometerkopf 11 vollständig durchläuft.

Das Disposable wird auf den Sockel 15 des Tonometerkörpers 11 aufgesteckt und bis zum Anschlag aufgeschoben. Das Disposable wird somit kraftschlüssig gehalten. Der Anschlag wird vorliegend durch einen Ringbeleuchtung 14 gebildet. Diese kontaktiert bei montiertem Disposable 20 den Rand desselben. Damit kann Licht von der Ringbeleuchtung 14 durch die Mantelflächen 23 und 22 hindurch bis zur Kontaktfläche 21 geleitet werden. Wird nun die Kontaktfläche 21 gegen eine Hornhaut eines Auges gedrückt, erscheint die mit der Hornhaut in Kontakt stehende Fläche dunkel. Die dunkle Fläche kann alsdann vermessen werden, um zum Beispiel nach Goldmann prüfen zu können, ob die vorgegebene Applanationsfläche erreicht ist. In der Anwendung wird anhand der ermittelten Applanationsfläche das Magnetfeld der Spulen in der Führungsvorrichtung 12 angesteuert, so dass ein Anpressdruck des Disposables 20 an der Hornhaut des Auges reguliert werden kann. Mittels einer Erfassungseinrichtung wird dazu fortlaufend die Applanationsfläche vermessen. Schliesslich wird die Kraft bestimmt, welche zur Applanation einer Fläche von 7.35 mm² respektive einer kreisrunden Fläche mit einem Durchmesser von 3.06 mm notwendig ist. Die Kraft kann aufgrund der Magnetfeldstärke errechnet werden. Weiter kann auch mit dem Positionssensor 13 aufgrund der Längsverschiebung des Tonometerkörpers 11 relativ zur Führungsvorrichtung 12 auf die Kraft rückgeschlossen werden.

Mittels des Aktuators, das heisst mittels der Führungsvorrichtung 12 und des Tonometerkopfes 11 kann nun der Tonometerkopf nicht nur linear bewegt werden, sondern auch in eine Schwingung versetzt werden. Die Eigenfrequenz ändert sich je nach Massenverteilung des Tonometerkopfes 11, so dass anhand der Eigenfrequenz ermittelt werden kann, ob das Tonometer 10 in einem Betriebsbereiten Zustand ist. Dazu kann eine Bandbreite der zulässigen Eigenfrequenz vorgegeben sein. Wenn der Messwert der Eigenfrequenz in der Bandbreite liegt, kann dem Tonometer ein betriebsbereiter Zustand zugeordnet werden, ansonsten kann zum Beispiel eine Warnung ausgegeben werden. Damit kann vermieden werden, dass das Disposable 20 nicht korrekt auf den Sockel aufgesteckt ist. Weiter kann vermieden werden, dass nicht geeignete Disposables eingesetzt werden. Nicht zuletzt kann damit verhindert werden, dass eine Messung gänzlich ohne Disposable durchgeführt wird.

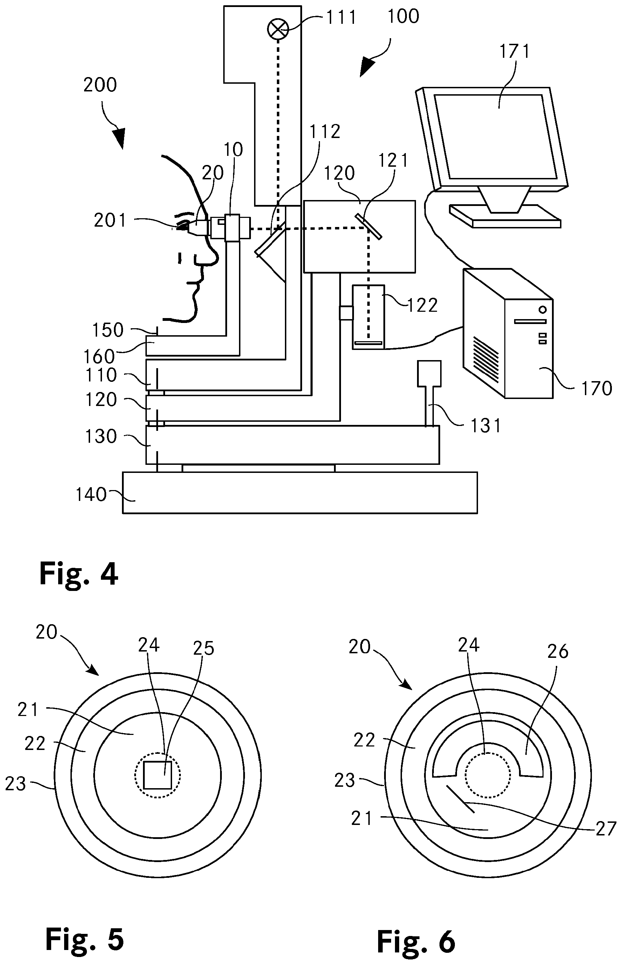

Die Figur 4 zeigt eine schematische Seitenansicht einer Spaltlampe 100 umfassend ein Tonometer 10.

Die Spaltlampe 100 umfasst einen Tonometerarm 160, eine Beleuchtungseinheit 110 und einen Optikteil 120 welche auf einem Kreuzschlitten 130 montiert sind. Der Kreuzschlitten 130 selbst ist auf einer Grundplatte 140 montiert, welche als Tischplatte ausgebildet sein kann, und kann in X-, Y- und Z-Richtung verfahren werden. Der Schlitten 130 ist über ein Bedienelement 131 steuerbar.

Die Spaltlampe 100 umfasst eine Hauptschwenkachse 150, über welche der L-förmige Tonometerarm 160, die Beleuchtungseinheit 110 und der Optikteil 120 schwenkbar gelagert sind. Der Tonometerarm 160 umfasst ein Tonometer 10, welches bei Bedarf in den Strahlengang der Spaltlampe 100 einschwenkbar ist.

Die Beleuchtungseinheit umfasst ein Leuchtmittel 111, welches über einen halbdurchlässigen Spiegel 112 Licht hin zu einem Auge 201 eines Patienten 200 leiten kann. Der Optikteil 120 umfasst einen Umlenkspiegel 121, womit eintretendes Licht sowohl in der Anwendung als Tonometer als auch in der Anwendung als Spaltlampe, vom Auge zu einem Bildsensor 122 geleitet werden kann. Der Bildsensor 122 ist wiederum mit einem Rechner, vorliegend ein separater Computer 170 verbunden ist, über welchen die Bilddaten des Bildsensor 122 ausgewertet werden können. Der Computer 170 ist mit einem Bildschirm 171 verbunden, an welchem die Bilddaten des Bildsensors 122 betrachtet werden können. Grundsätzlich können auch mehr als ein Bildsensor vorgesehen sein, womit zum Beispiel unterschiedliche Spektren (UV, IR etc.) abgedeckt werden können.

Mit dem Bildsensor 122 kann nun nicht nur die Applanationsfläche ermittelt werden, sondern auch ein Zustand des Disposables 20 überprüft werden.

Insbesondere kann durch eine Bildanalyse zum Beispiel ermittelt werden, ob die Kontaktfläche sauber und frei von Beschädigungen ist, so dass eine korrekte Messung des Augeninnendruckes erreicht werden kann.

Im Verfahren wird nach dem Montieren des Disposables 20 am Tonometerkörper 11 und vor der Durchführung der tonometrischen Messung mit dem Bildsensor 122 mindestens ein Bild aufgenommen und analysiert. Dabei kann zum Beispiel festgestellt werden, ob die Kontaktfläche Kratzer, Risse, Brüche, Verunreinigungen etc. aufweist. Aufgrund des Befundes kann dem Tonometer ein Betriebszustand zugeordnet werden. Sofern die Beschädigungen zu gross sind, kann eine Warnung ausgegeben oder das Tonometer elektronisch blockiert werden, so dass keine Messungen durchgeführt werden können. Alternativ kann bei geringfügigen Beschädigungen oder Unregelmässigkeiten eine Kalibration durchgeführt werden, so dass die anschliessende tonometrische Messung hinsichtlich der Beschädigungen oder Unregelmässigkeiten bereinigt werden können.

Sofern keine Beschädigungen oder Verschmutzungen ermittelt werden, kann direkt ein normaler Betriebszustand zugeordnet werden, worauf die tonometrische Messung durchgeführt werden kann.

Weiter kann mit dem Bildsensor 122 auch ermittelt werden, ob weiter Teile des Tonometers 10, insbesondere die optischen Teile, Verunreinigungen oder Beschädigungen aufweisen. Zum Beispiel kann eine Oberfläche des Sockels 15 auf Verunreinigungen oder Beschädigungen überprüft werden.

In einer weiteren Ausführungsform wird nacheinander oder gleichzeitig eine Prüfung des korrekten Sitzes und des korrekten Typs des Disposables mittels Erzeugung einer Schwingung mit der in der Führungsvorrichtung 12 enthaltenen Spulen sowie dem magnetischen Element des Tonometerkörpers 11 und eine Prüfung des Zustandes der Kontaktfläche des Disposables durchgeführt. Damit wird ein besonders sicheres Verfahren zur tonometrischen Messung erreicht.

Die Figur 5 zeigt eine schematische Draufsicht auf ein Disposable 20 mit einer zentral auf der Kontaktfläche 21 aufgebrachtem Code 25. Die Blickrichtung ist dabei in die Öffnung des Disposables 20 gerichtet.

Die Kontaktfläche 21 des Disposables 20 kommt in der Praxis lediglich mit einem weitgehend vorbekannten Kontaktbereich 24 in Kontakt mit der Hornhaut. Bei einem Verfahren nach Goldmann weist die Applanationsfläche eine Fläche von 7.35 mm² auf und ist im Wesentlichen kreisförmig. Der Bereich im Innern dieser Fläche ist nicht von Interesse, da zur Bestimmung der Fläche lediglich die Kontur der Fläche vermessen wird. Typischerweise werden ein oder mehrere Durchmesser ermittelt, um schliesslich die Fläche zu berechnen. Der Innere Bereich dieser Fläche kann nun zum Aufdrucken oder Gravieren von Informationen verwendet werden. Vorliegend ist nun innerhalb des Bereiches ein QR-Code 25 vorgesehen, welcher durch den Bildsensor 122 erfasst und mittels einer Recheneinheit 170 ausgewertet werden kann. Der QR-Code umfasst Informationen, über welche das Disposable identifiziert werden kann. Vorliegend umfassen die Informationen den Hersteller, das Herstellungsdatum, das Verfalldatum, eine Chargennummer und eine Seriennummer. Insbesondere anhand der Seriennummer kann ermittelt werden, ob das Disposable neu ist, das heiss, bisher für keine Messung eingesetzt worden ist.

Die Figur 6 zeigt als weitere Variante eine schematische Draufsicht auf ein Disposable 20 mit einem im Randbereich der Kontaktfläche 21 aufgebrachtem Code 26. Der Code 26 ist ausserhalb der für die tonometrische Messung notwendige Bereich der Kontaktfläche 21 angeordnet. Der Code 26 weist die Form eines Halbkreisrings auf, kann aber auch eine Form eines Kreisrings aufweisen, welcher den Code 26 vollständig umschliesst. Der Code 26 ist vorliegend als zirkularer Strichcode ausgebildet und umfasst dieselben Informationen, wie der obig beschriebene QR-Code 26. Der Code 26 wird ebenfalls im Verfahren durch den Bildsensor 122 aufgenommen und durch den Computer 170 ausgewertet, so dass das Disposable 20 identifiziert werden kann.

Weiter umfasst die Kontaktfläche 21 des Disposables 20 eine Referenzgrösse 28 in Form einer Linie mit definierter Länge. Damit kann das Gerät kalibriert werden.

Der Bildsensor muss nicht zwingend durch eine Spaltlampe zur Verfügung gestellt sein. Der Bildsensor kann auch direkt durch das Tonometer selbst oder durch ein anderweitiges Untersuchungsgerät umfasst sein.

Die Recheneinheit respektive der Computer muss nicht als autonomes Gerät ausgebildet sein, sondern kann Teil der Spaltlampe sein.

Statt dem elektromagnetischen Aktuator kann auch ein Linearantrieb oder andere dem Fachmann bekannt Mittel zum Verfahren des Tonometerkopfes vorgesehen sein.

Das Disposable kann aber auch aus anderen transparenten Kunststoffen, Glas oder dergleichen hergestellt sein. Das Disposable muss nicht zwingend kappenförmig ausgebildet sein, sondern kann zum Beispiel auch zylindrisch, insbesondere aus Vollmaterial etc. ausgebildet sein. In diesem Fall kann der Tonometerkörper statt des Sockels auch eine Zylindrische Vertiefung umfassen, in welche das Disposable eingesteckt werden kann. Das Disposable muss nicht zwingend kraftschlüssig gehalten sein, sondern kann auch formschlüssig oder form- und kraftschlüssig gehalten sein. Dem Fachmann sind dazu beliebig viele Möglichkeiten bekannt, wie zum Beispiel ein Bajonettverschluss, Schraubverschluss etc.

Zusammenfassend ist festzustellen, dass erfindungsgemäss ein Verfahren zum Prüfen eines Disposables geschaffen wird, womit ein medizinisches Untersuchungsverfahren, insbesondere ein tonometrisches Messverfahren sicherer und robuster durchführbar ist.

## Patentansprüche

1. Verfahren zum Prüfen eines Disposables (20) an einem medizinischen Untersuchungsgerät, insbesondere an einem Tonometer (10), wobei das medizinische Untersuchungsgerät
- eine Disposable-Haltevorrichtung (15) umfasst, an welchem ein Disposable (20) gehalten und insbesondere zur applanationstonometrischen Messung mit einem Patientenauge in Kontakt bringbar ist;
- eine Erfassungseinrichtung (11, 12; 122) umfasst, womit eine physikalische Eigenschaft des Disposables (20) gemessen werden kann;
**dadurch gekennzeichnet, dass**
- mittels der Erfassungseinrichtung (11, 12; 122) ein Messwert der physikalischen Eigenschaft des Disposables ermittelt wird.

2. Verfahren nach Anspruch 1, **dadurch gekennzeichnet, dass** aufgrund des Messwertes dem medizinischen Untersuchungsgerät, insbesondere dem Tonometer (10) ein Betriebszustand zugeordnet wird.

3. Verfahren nach Anspruch 1 oder 2, **dadurch gekennzeichnet, dass** die physikalische Eigenschaft eine Eigenfrequenz des Disposables (20) und/oder eines das Disposable (20) umfassenden Teils des medizinischen Untersuchungsgeräts, insbesondere des Tonometers (10) umfasst.

4. Verfahren nach Anspruch 3, **dadurch gekennzeichnet, dass** zur Ermittlung der Eigenfrequenz das Disposables (20) und/oder ein das Disposable (20) umfassender Teil des medizinischen Untersuchungsgeräts, insbesondere des Tonometers (10), durch eine Vorrichtung zur Erzeugung einer Applanationskraft (11, 12), in eine Schwingung versetzt wird.

5. Verfahren nach Anspruch 3 oder 4, **dadurch gekennzeichnet, dass** die Schwingung elektromagnetisch erzeugt wird.

6. Verfahren nach einem der Ansprüche 3 bis 5, **dadurch gekennzeichnet, dass** anhand des Messwertes der Eigenfrequenz mindestens eines der folgenden Merkmale bestimmt wird:
- eine Ausrichtung des Disposables (20) am medizinischen Untersuchungsgerät;
- eine Masse des Disposables (20);
- eine Massenverteilung, insbesondere ein Schwerpunkt des Disposables (20).

7. Verfahren nach einem der Ansprüche 1 bis 6, **dadurch gekennzeichnet, dass** die physikalische Eigenschaft ein optisches Reflexionsverhalten des Disposables (20) umfasst.

8. Verfahren nach Anspruch 7, **dadurch gekennzeichnet, dass** das optische Reflexionsverhalten ein optisches Erfassen von Daten des Disposables (20) umfasst.

9. Verfahren nach Anspruch 8, **dadurch gekennzeichnet, dass** die Daten des Disposables (20) einen Code (25, 26), insbesondere einen QR-Code (25) oder einen Barcode (26) umfassen, wobei mit dem medizinischen Untersuchungsgerät, insbesondere dem Tonometer und/oder einer Spaltlampe der Code erfasst wird.

10. Verfahren nach einem der Ansprüche 7 bis 9, **dadurch gekennzeichnet, dass** Verunreinigungen des Disposables (20) aufgrund des optischen Reflexionsverhaltens ermittelt werden.

11. Verfahren nach einem der Ansprüche 6 bis 10, **dadurch gekennzeichnet, dass** das optische Reflexionsverhalten mit einem Bildsensor (122) des Untersuchungsgeräts, insbesondere des Tonometers, und/oder einer Spaltlampe (100), ermittelt wird.

12. Prüfkörper (20) zur Verwendung in einem Tonometer, wobei der Prüfkörper (20) eine Kontaktfläche (21) zum Kontaktieren der Hornhaut umfasst, **dadurch gekennzeichnet, dass** der Prüfkörper (20) einen Code umfasst, welcher insbesondere durch eine Erfassungseinrichtung des Tonometers (20) und/oder einer Spaltlampe (100) erfassbar ist.

13. Prüfkörper(20) nach Anspruch 12, **dadurch gekennzeichnet, dass** die Kontaktfläche (21) einen Code (25, 26) umfasst, welcher durch eine Erfassungseinrichtung des Tonometers (10) und/oder einer Spaltlampe (100) erfassbar ist.

14. Prüfkörper (20) nach Anspruch 12 oder 13, **dadurch gekennzeichnet, dass** der Code (25, 26) zentral auf der Kontaktfläche (21), insbesondere auf einer Fläche kleiner als 3x3 mm, besonders bevorzugt kleiner als 2x2 mm, insbesondere bevorzugt auf ungefähr 1.5x1.5 mm dargestellt ist.

15. Prüfkörper (20) nach einem der Ansprüche 12 oder 13, **dadurch gekennzeichnet, dass** der Code (26) kreisringförmig ausgebildet und in einem zur Kontaktfläche (21) peripheren Bereich angeordnet ist.

16. Vorrichtung zur Durchführung eines Verfahrens nach einem der Ansprüche 1 bis 11, umfassend ein Tonometer mit einer Disposable-Haltevorrichtung, **dadurch gekennzeichnet, dass** die Disposable-Haltevorrichtung eine Ringbeleuchtung umfasst, welche als Anschlag für einen Rand eines kappenförmigen Disposables ausgebildet ist.
